Europäisches Patentamt

European Patent Office

Office européen des brevets

(11) Numéro de publication: **0 316 494**
**A1**

## (12) DEMANDE DE BREVET EUROPEEN

(21) Numéro de dépôt: 87402612.3

(22) Date de dépôt: **19.11.87**

(51) Int. Cl.⁴: **C13F 1/02 , B01D 9/00**

(43) Date de publication de la demande:
**24.05.89 Bulletin 89/21**

(84) Etats contractants désignés:
**AT BE DE ES GB IT NL**

(71) Demandeur: **FIVES-CAIL BABCOCK**
**38, rue de la République**
**F-93107 Montreuil Cédex(FR)**

(72) Inventeur: **Chielens, Alain**
**37, rue de la Briqueterie**
**F-59420 Mouvaux(FR)**
Inventeur: **Journet, Gérard**
**9, rue de l'Houssoye**
**F-59310 Coutiches(FR)**
Inventeur: **Werquin, Pierre**
**10, rue de Valmy**
**F-59700 Marcq en Baroeul(FR)**

(74) Mandataire: **Fontanié, Etienne**
**FIVES-CAIL BABCOCK 38, rue de la**
**République**
**F-93107 Montreuil Cédex(FR)**

(54) **Procédé de conduite automatisée d'un appareil de cristallisation à marche continue pour la production de sucre.**

(57) L'invention a pour objet un procédé de conduite automatisée d'un appareil de cristallisation à marche continue (20) pour la production de sucre selon le brevet EP-0.162.739.

Pour permettre une auto-adaptation du modèle mathématique utilisé pour cette conduite aux conditions réelles du fonctionnement de l'appareil, les valeurs d'un ou plusieurs paramètres (coefficient d'échange thermique et / ou surface d'échange thermique du faisceau de chauffage) utilisés dans les calculs de bilan thermique des différents compartiments (1, 2, ... n) sont déterminés à chaque pas de calcul en fonction de la valeur calculée (par 32) au pas de calcul précédent et de la valeur mesurée (en 56) du débit d'eau condensée dans le faisceau de chauffage (12).

EP 0 316 494 A1

FIG. 1

# PROCEDE DE CONDUITE AUTOMATISEE D'UN APPAREIL DE CRISTALLISATION A MARCHE CONTINUE POUR LA PRODUCTION DE SUCRE

La présente invention a pour objet un procédé de conduite automatisée d'un appareil de cristallisation à marche continue pour la production de sucre, constitué par un cuve fermée, divisée par des cloisons en plusieurs compartiments munis d'un faisceau de chauffage, une solution sucrée étant introduite dans chacun des compartiments, un magma formé d'un mélange de petits cristaux et de liqueur-mère étant introduit dans le premier compartiment et la masse cuite produite étant extraite du dernier compartiment.

Selon l'une des formes de mise en oeuvre du procédé objet du brevet EP-0.162.739, on détermine périodiquement la valeur optimale d'un premier paramètre, par exemple le débit d'alimentation en solution sucrée de chaque compartiment, en utilisant une loi préétablie donnant la valeur dudit paramètre dans les compartiments en fonction du brix de la solution à traiter, de la pression de vapeur dans la calandre de l'appareil et du rapport

$R = (QMAG \times RDTMAG) / (QEGT \times BEGT)$ avec

QMAG : débit de magma

RDTMAG : teneur en cristaux du magma

QEGT : débit total de solution sucrée à traiter

BEGT : brix de la solution sucrée à traiter,

on calcule la valeur optimale d'un second paramètre, qui peut être la pression de vapeur de chauffage, soit le débit total de solution à traiter, en utilisant pour ce calcul les valeurs imposées du débit total de solution sucrée ou de la pression de vapeur de chauffage, respectivement, et du brix de la masse cuite produite et éventuellement un terme correctif dont la valeur est fonction de l'écart entre la valeur imposée du brix de la masse cuite et la valeur mesurée de ce brix, et on règle ces paramètres pour les maintenir à des valeurs de consigne égales aux valeurs optimales calculées.

Le but de la présente invention est d'apporter à ce procédé des perfectionnements permettant une auto-adaptation du modèle mathématique utilisé pour les calculs aux conditions réelles de fonctionnement de l'appareil.

Suivant une première caractéristique de l'invention, les valeurs d'un ou plusieurs paramètres utilisés dans les calculs sont déterminées, à chaque pas de calcul, en fonction de la valeur calculée au pas de calcul précédent et de la valeur mesurée du débit d'eau condensée dans le faisceau de chauffage.

On effectue ainsi, à chaque pas de calcul, une correction des paramètres utilisée pourle calcul, ce qui permet notamment de prendre en compte l'encrassement progressif des éléments du faisceau de chauffage entre deux nettoyages. Cette correction peut porter sur les coefficients d'échange thermique et/ou les surfaces d'échange thermique utilisés pour le calcul des bilans thermiques. Les coefficients d'échange thermique peuvent, par exemple, être déterminés au moyen de la formule :

$$h = ho \left(a + b . \frac{QCOND + QVAP}{2QNOM}\right)$$

dans laquelle QCOND est le débit mesuré d'eau condensée dans le faisceau de chauffage, QVAP est le débit d'eau condensée calculé au pas de calcul précédent, QNOM est le débit total d'eau condensée correspondant à la valeur ho du coefficient d'échange et a et b sont des constantes propres à chaque compartiment ou groupe de compartiments.

Pour le calcul des surfaces d'échange effectives ou utiles on pourra utiliser la formule

surface échange effective = surface d'échange théorique . FXj

$$\text{avec } FXj = 1 - \frac{1 - XENCRA}{1 - FXDn} . (1 - FXDj)$$

n étant le nombre de compartiments, j l'induce du compartiment considéré, FXDj une constante caractéristique du compartiment et XENCRA une grandeur fonction de l'écart entre la valeur mesurée QCOND et la valeur calculée QVAP du débit total d'eau condensée. XENCRA peut, par exemple, être élaborée par un régulateur de type PID recevant, en entrée, les valeurs mesurée et calculée du débit d'eau condensée.

Selon une autre caractéristique de l'invention, on passe automatiquement d'un mode de conduite avec détermination de la valeur optimale de la pression de la vapeur de chauffage, le débit total de solution à traiter étant maintenu à une valeur prédéterminée, à un mode de conduite avec détermination de la valeur optimale du débit total de solution à traiter, la pression de la vapeur de chauffage étant imposée, lorsque la valeur optimale de la pression de vapeur de chauffage est supérieure à la pression de vapeur disponible, et on revient au premier mode de conduite lorsque la pression de vapeur disponible devient supérieure à la valeur imposée de la pression de la vapeur de chauffage.

La description qui suit d'un mode de mise en oeuvre de l'invention, donné à titre d'exemple non limitatif, se réfère aux dessins qui l'accompagnent et sur lesquels :

La figure 1 est le schéma d'un système de conduite automatique d'un appareil de critallisation pour la mise en oeuvre du procédé de l'invention, et

La figure 2 est l'organigramme du procédé.

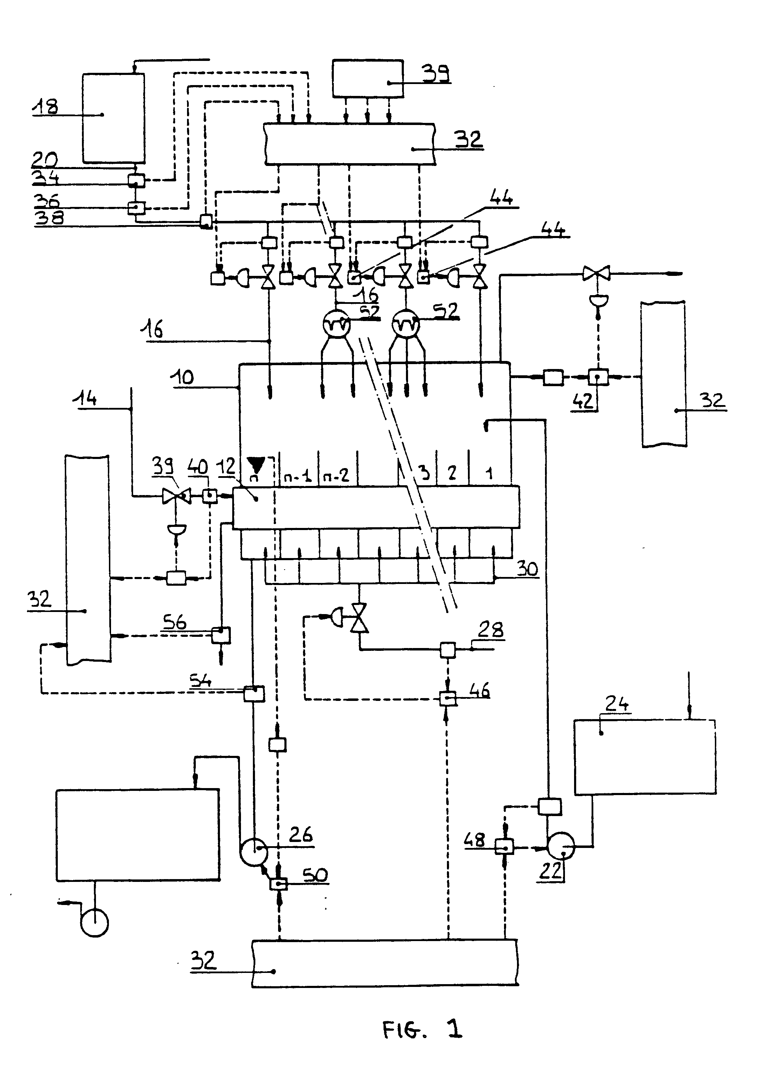

Sur la figure 1, le numéro de référence 10 désigne un appareil de cristallisation par évaporation à marche continue constitué par une cuve fermée disposée horizontalement et divisée par des cloisons verticales en plusieurs compartiments 1, 2, 3, 4 ..., n-2, n-1, n. Ces cloisons n'atteignent pas le sommet de la cuve de sorte que tous les compartiments communiquent entre eux à la partie supérieure de la cuve. Des ouvertures sont également prévues dans les cloisons pour permettre le passage de la masse cuite d'un compartiment à l'autre. Dans la partie inférieure de la cuve est placé un faisceau de chauffage 12 commun à tous les compartiments et formé de tubes ou de plaques dans lesquels on fait circuler de la vapeur amenée par une conduite 14.

Une solution sucrée, qui sera appelée égout dans ce qui suit, est distribuée dans les différents compartiments par des tuyauteries 16 ; cet égout provient d'un bac 18 auquel les tuyauteries 16 sont reliées par une conduite 20. On peut prévoir une tuyauterie d'alimentation par compartiment ou une tuyauterie commune à un groupe de compartiments. Dans ce cas, la répartition du débit entre les compartiments du groupe pourra être faite par exemple au moyen d'un dispositif 52 du type faisant l'objet du brevet français 84.00101.

Un magma constitué par un mélange de petits cristaux constituant des germes de cristallisation et de liqueur-mère est introduit dans le premier compartiment au moyen d'une pompe à débit variable 22 prélevant le magma sur une capacité 24.

La masse cuite produite est extraite du dernier compartiment au moyen d'un pompe à débit variable 26.

De la vapeur est injectée à la partie inférieure de chaque compartiment, pour agiter la masse cuite et favoriser les échanges thermiques ; cette vapeur amenée par une conduite 28 est distribuée à des injecteurs 30 placés dans le bas des compartiments.

Le numéro de référence 32 désigne un système de conduite automatisée se composant d'un ordinateur de supervision couplé à un système de conduite de processus comprenant les régulateurs numériques et l'instrumentation ou à des chaines de régulation numériques déportées, ou à des chaines de régulation analogiques classiques. En variante l'ordinateur peut assurer les fonctions de régulation et communiquer avec l'instrumentation par des convertisseurs digital-analogique et analogique-digital. Le système reçoit des informations numériques et analogiques, les traite et émet les consignes qu'il a optimisées vers les actionneurs : vannes, moteurs, etc... Pour simplifier la figure 1, les différents éléments constitutifs de ce système ont été répartis sur le dessin.

Le système 32 reçoit en permanence des informations sur la température, le débit total et le brix de l'égout qui sont mesurées par des instruments 34, 36 et 38, respectivement, et des informations sur le brix de la masse cuite fournies par un instrument 54. Il reçoit en outre des données externes : résultats d'analyses de laboratoire, caractéristiques imposées par l'exploitant, etc... qui sont introduites par exemple au moyen d'un clavier 39. Il a également en mémoire les caractéristiques technologiques de l'appareil 10 : dimensions et volume utile de la cuve, structure du faisceau de chauffage, dimension des tubes du faisceau, etc...

Le système comprend en outre un régulateur de la pression de la vapeur de chauffage 40, un régulateur 42 de la pression de vapeur dans la calandre de l'appareil, des régulateurs de débit partiel d'égout 44, un régulateur de débit de vapeur d'agitation 46, un régulateur de débit de magma 48 commandant la pompe 22 et un régulateur de niveau de masse cuite 50 commandant la pompe 26.

Conformément à l'invention du brevet EP-0.162.739, on détermine à intervalles de temps réguliers les valeurs optimales des débits d'alimentation en égout de chaque compartiment ou groupe de compartiments, du débit de magma admis dans le premier compartiment, de la pression de la vapeur de chauffage et du débit de vapeur d'agitation injecté dans les compartiments et on les utilise comme valeurs de consigne dans les régulateurs correspondants. Ces intervalles de temps pourront être, par exemple, de 15 secondes. Les valeurs optimales des débits d'alimentation QEGj des compartiments, du débit de magma QMAG et du débit de vapeur d'agitation QINJ sont déterminées comme décrit dans le brevet mentionné ci-dessus.

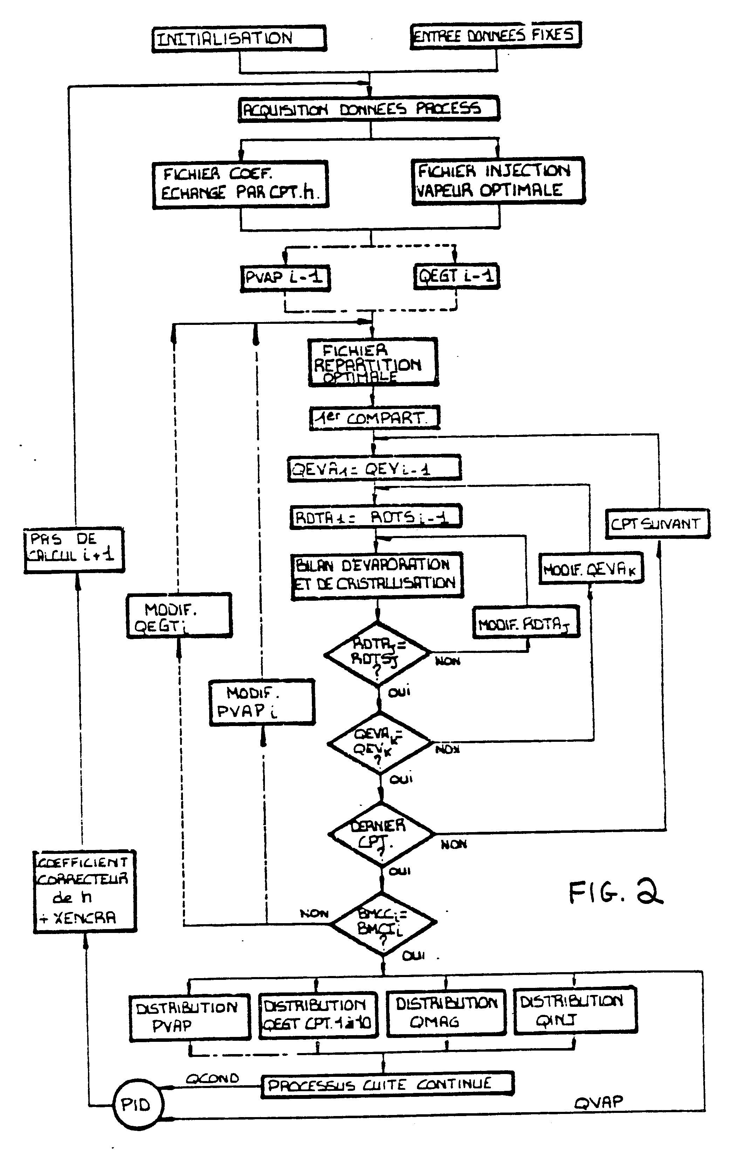

La valeur optimale de la pression de la vapeur de chauffage PVAP est calculée par le système 32 suivant le procédé présenté sous forme d'organigramme sur la figure 2. Le système 32 effectue pour chaque compartiment un bilan de cristallisation et un bilan thermique d'eau évaporée en utilisant les valeurs mesurées ou déterminées comme indiqué plus haut des différentes grandeurs caractérisant l'égout à traiter et le magma (débit, brix, température, etc...), ainsi que celles des différents paramètres caractérisant le fonctionnement de l'appareil (débits d'alimentation en égout, débit de vapeur d'agitation, pression de vapeur dans la calandre de l'appareil, niveau de masse cuite dans l'appareil, etc...) comme décrit au brevet principal.

Pour effectuer les bilans thermiques, le système 32 utilise la formule suivante :

$$QEVj = (QMCE \times EhMCE + YEGj \times QEGT \times EhEGT - QMCS \times EhMCS$$
$$- \text{Pertes thermiques} + h \times \Delta S \times \Delta tu) / H$$

dans laquelle QEVj est la quantité d'eau évaporée dans le compartiment j, QMCE et QMCS étant, respectivement, les débits de la masse cuite entrant dans le compartiment et sortant du compartiment, YEGj étant le débit d'alimentation en égout du compartiment rapporté au débit total d'égout, EhMCE, EhMCS et EhEGT étant, respectivement, l'enthalpie spécifique de la masse cuite entrant dans le compartiment, de la masse cuite sortant du compartiment et de l'égout, h étant le coefficient d'échange thermique, $\Delta S$ la surface d'échange thermique effective du compartiment, $\Delta tu$ l'écart de température utile, et H l'enthalpie spécifique de la vapeur de chauffage.

Pour le premier compartiment les valeurs de QMCE et EhMCE sont celles des grandeurs correspondantes du magma.

Conformément à la présente invention, les coefficients d'échange thermique sont donnés par la formule

$$h = ho \cdot (a + b \frac{QCOND + QVAP}{2 \, QNOM})$$

et leur valeur est déterminée à chaque pas de calcul en fonction du débit d'eau condensée QCOND, mesuré au moyen du débitmètre 56, et du débit d'eau condensée QVAP, calculé par le système 32, a et b étant des constantes propres à chaque compartiment ou groupe de compartiments et ho le coefficient d'échange thermique théorique correspondant à des conditions de fonctionnement prises pour référence (débit nominal, appareil propre...) et pour lesquelles le débit d'eau condensée est égal à QNOM. Les valeurs de a, b, ho et QNOM sont stockées dans un fichier du système 32.

La surface d'échange thermique effective utilisée pour le calcul est donnée par la formule
$$\Delta S = \Delta S \text{ théorique} \cdot FXj$$

$$avec \quad FXj = 1 - \frac{1 - XENCRA}{1 - FXDn} \cdot (1 - FXDj)$$

Les valeurs théoriques des surfaces d'échange thermique ($\Delta S$ théorique) des différents compartiments sont calculées initialement à partir des données géométriques (diamètre et épaisseur des tubes, écartement des tubes du faisceau...) et stockées dans un fichier du système 32. Les constantes FXDj sont également stockées dans un fichier.

Le coefficient XENCRA permet de tenir compte de l'encrassement des tubes du faisceau. Sa valeur est celle du signal de sortie d'un régulateur numérique de type PID, incorporé au système 32 et à l'entrée duquel sont appliqués des signaux représentant les valeurs QCOND (débit d'eau condensée mesurée par le débitmètre 56) et QVAP (débit d'eau condensée calculée). La valeur de XENCRA est donc fonction de l'écart entre les valeurs de QCOND et QVAP.

Le système effectue un bilan de cristallisation et un bilan thermique d'eau évaporée pour chacun des compartiments, du premier au dernier, en utilisant comme valeurs des grandeurs caractéristiques de la masse cuite entrant dans un compartiment les valeurs calculées pour la masse cuite sortant du compartiment précédent.

Lorsque les bilans ont été bouclés pour tous les compartiments, on compare la valeur calculée du brix BMCC à la valeur imposée BMCI. Si ces deux valeurs sont égales, à la précision recherchée près, on arrête le calcul et on retient pour valeur optimale de la pression de vapeur de chauffage la valeur PVAPi-1 attribuée par le système au début du calcul. Si non, on remplace cette valeur par une nouvelle valeur calculée suivant une loi déterminée en fonction de l'écart entre BMCI et BMCC, et on recommence tous les calculs en utilisant cette nouvelle valeur. Si le brix BMCC calculé à partir de cette nouvelle valeur ne satisfait pas l'égalité BMCC = BMCI on recommence l'ensemble des calculs après avoir à nouveau modifié la valeur de PVAP. Lorsque cette égalité est vérifiée avec l'approximation désirée, les calculs sont arrêtés et la dernière valeur de PVAP retenue comme valeur optimale de la pression de la vapeur de chauffage et utilisée comme valeur de consigne du régulateur 40.

Le débit d'eau condensée QVAP calculé par le système 32 à chaque pas de calcul est utilisé, avec le débit d'eau condensée QCOND mesuré par le débitmètre 56, pour déterminer les nouvelles valeurs des coefficients d'échange thermique h et du coefficient XENCRA, et celles-ci sont utilisées au pas de calcul suivant.

Le fonctionnement décrit ci-dessus, qui consiste à imposer un débit total d'égout et à régler la pression de la vapeur de chauffage pour la maintenir à la valeur optimale calculée par le système 32 est le fonctionnement normal. Cependant, lorsque la pression sous laquelle la vapeur de chauffage est disponible n'est pas suffisante, la vanne 39 commandée par le régulateur 40 s'ouvre en grand sans que l'on puisse amener la pression à la valeur optimale calculée. Conformément à l'invention, le système 32 maintient alors la pression de la vapeur de chauffage constante et inférieure ou au plus égale à la pression maximale sous laquelle la vapeur peut être fournie et calcule la valeur optimale du débit total d'égout alimentant l'appareil 10 puis, au moyen d'une loi de répartition préétablie, les débits d'alimentation en égout des différents compartiments, les valeurs calculées étant imposées comme consignes aux régulateurs de débit partiels 44, pour maintenir le brix de la masse cuite produite à la valeur imposée. Le passage du premier mode de conduite au second mode s'effectue automatiquement lorsque le degré d'ouverture de la vanne 39 dépasse une valeur prédéterminée, par exemple 80% . Le retour du premier mode de conduite s'effectue lorsque le degré d'ouverture de la vanne 39 redevient inférieur à une seconde valeur prédéterminée, inférieure à la première, par exemple 70%.

**Revendications**

1. Procédé de conduite automatisée d'un appareil de cristallisation à marche continue pour la production de sucre, constitué par une cuve fermée, divisée par des cloisons en plusieurs compartiments munis d'un faisceau de chauffage, une solution sucrée étant introduite dans chacun des compartiments, un magma formé d'un mélange de petits cristaux et de liqueur mère étant introduit dans le premier compartiment et la masse-cuite produite étant extraite du dernier compartiment, procédé selon lequel on calcule périodiquement la valeur optimale de la pression de vapeur de chauffage (PVAP) ou le débit total de solution à traiter (QEGT) en effectuant un bilan de cristallation et un bilan thermique d'eau évaporée pour chacun des compartiments et on maintient la pression de la vapeur de chauffage ou le débit total de solution à traiter à la valeur optimale calculée, caractérisé en ce que les valeurs d'un ou plusieurs paramètres (h, $\Delta$ S) utilisés dans les calculs de bilan thermique des différents compartiments sont déterminées à chaque pas de calcul en fonction de la valeur calculée au pas de calcul précédent (QVAP) et de la valeur mesurée (QCOND) de débit d'eau condensée dans le faisceau de chauffage.

2. Procédé selon la revendication 1, caractérisé en ce que ledit paramètre est le coefficient d'échange thermique à travers les éléments du faisceau de chauffage (h).

3. Procédé selon la revendication 1 ou 2, caractérisé en ce que ledit paramètre est la surface d'échange thermique des éléments du faisceau de chauffage ($\Delta$ S).

4. Procédé selon la revendication 2, caractérisé en ce que le coefficient d'échange thermique (h) est déterminé au moyen de la formule :

$$h = ho \left( a + b \cdot \frac{QCOND + QVAP}{2\ QNOM} \right.$$

dans laquelle ho, a et b sont des constantes propres à chaque compartiment et QNOM est une constante représentant le débit d'eau condensée produit quand h = ho.

5. Procédé selon la revendication 3, caractérisé en ce que la surface d'échange thermique ($\Delta$ S) est déterminée au moyen des formules

$\Delta$ S = $\Delta$ S théorique x FXj

et

$$FXj = 1 - \frac{1 - XENCRA}{1 - FXDn} (1 . FXDj)$$

dans lesquelles $\Delta$ S théorique et FXDj sont des constantes propres à chaque compartiment et XENCRA est un coefficient correcteur qui est fonction de l'écart entre la valeur mesurée (QCOND) et la valeur calculée (QVAP) du débit d'eau condensée.

6. Procédé selon la revendication 5, caractérisé en ce que la valeur dudit coefficient correcteur (XENCRA) est celle du signal de sortie d'un régulateur de type PID à l'entrée duquel sont appliqués des signaux proportionnels aux valeurs mesurées et calculées (QCOND, QVAP) du débit d'eau condensée.

7. Procédé selon l'une quelconque des revendications précédentes, caractérisé en ce qu'on calcule la valeur optimale de la pression de la vapeur de chauffage (PVAP) et on maintient la pression de la vapeur de chauffage à la valeur optimale calculée, le débit total de solution à traiter (QEGT) étant maintenu à une valeur prédéterminée, tant que ladite valeur optimale est inférieure à la pression de vapeur disponible, et on calcule la valeur optimale du débit total de solution à traiter et on maintient ce débit à la valeur optimale calculée, la pression de vapeur de chauffage étant maintenue à une valeur imposée, tant que la pression de vapeur disponible reste inférieure à une pression prédéterminée.

FIG. 1

FIG. 2

Office européen
des brevets

**RAPPORT DE RECHERCHE EUROPEENNE**

Numero de la demande

EP 87 40 2612

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | FR-A-2 562 908 (FIVES-CAIL BABCOCK) <br> * Revendications 1-9; figure 2 * & <br> EP-A-162 739 <br> --- | 1-7 | C 13 F 1/02 <br> B 01 D 9/00 |
| A | FR-A-2 101 257 (FIVES-CAIL BABCOCK) <br> * Revendications 1-7 * <br> --- | 1-7 | |
| A | US-A-4 155 774 (RANDOLPH) <br> * Revendications 1,2 * <br> --- | 1 | |
| A | US-A-4 263 010 (RANDOLPH) <br> * Revendications 1,19 * <br> --- | 1 | |
| A | AU-B- 520 244 (SKYRING) <br> * Revendications 1-21 * <br> ----- | 1 | |

**DOMAINES TECHNIQUES RECHERCHES (Int. Cl.4)**

C 13 F
C 13 G
B 01 D

Le présent rapport a été établi pour toutes les revendications

| Lieu de la recherche | Date d'achèvement de la recherche | Examinateur |
|---|---|---|
| LA HAYE | 25-07-1988 | VAN MOER A.M.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

................................................................
& : membre de la même famille, document correspondant

EPO FORM 1503 03.82 (P0402)